# EUROPEAN PATENT SPECIFICATION

(11) **EP 3 142 661 B1**
(45) Date of publication and mention of the grant of the patent: **06.10.2021**
(21) Application number: 15726257.7
(22) Date of filing: 14.05.2015
(51) Int. Cl.: A61K 31/40, A61K 31/403, A61K 31/4985, A61K 31/522, A61K 31/70, A61K 31/7042, A61P 3/10, A61K 38/28, A61K 31/155

(54) **METHOD FOR SUPPRESSING GLUCAGON SECRETION OF AN SGLT2 INHIBITOR**
VERFAHREN ZUR UNTERDRÜCKUNG DER GLUCAGONSEKRETION EINES SGLT2-HEMMERS
MÉTHODE DE SUPPRESSION DE LA SÉCRÉTION DE GLUCAGON D'UN INHIBITEUR DE SGLT2

(30) Priority: 16.05.2014 US 201461994222 P; 12.06.2014 US 201462011084 P
(43) Date of publication of application: 22.03.2017
(73) Proprietor: Astrazeneca AB, 151 85 Södertälje (SE)
(72) Inventor: HIRSHBERG, Boaz, Gaithersburg MD 20878 (US); IQBAL, Nayyar, Gaithersburg MD 20878 (US)
(74) Representative: AstraZeneca Intellectual Property
(86) International application number: PCT/GB2015/051437
(87) International publication number: WO 2015/173584

(56) References cited:
- WO-A1-2009/091082
- WO-A1-2010/092125
- WO-A1-2014/161918
- US-B2- 6 515 117
- US-B2- 6 774 112
- JABBOUR SERGE A ET AL: "Dapagliflozin is effective as add-on therapy to sitagliptin with or without metformin: a 24-week, multicenter, randomized, double-blind, placebo-controlled study.", DIABETES CARE MAR 2014, vol. 37, no. 3, March 2014 (2014-03), pages 740-750, XP002742649, ISSN: 1935-5548
- ZAMBROWICZ BRIAN ET AL: "Effects of LX4211, a Dual SGLT1/SGLT2 Inhibitor, Plus Sitagliptin on Postprandial Active GLP-1 and Glycemic Control in Type 2 Diabetes", CLINICAL THERAPEUTICS, vol. 35, no. 3, March 2013 (2013-03), pages 273-285.e7, XP002742650, DOI: 10.1016/j.clinthera.2013.01.010
- HANSEN LARS ET AL: "Postprandial dynamics of plasma glucose, insulin, and glucagon in patients with type 2 diabetes treated with saxagliptin plus dapagliflozin add-on to metformin therapy.", ENDOCRINE PRACTICE : OFFICIAL JOURNAL OF THE AMERICAN COLLEGE OF ENDOCRINOLOGY AND THE AMERICAN ASSOCIATION OF CLINICAL ENDOCRINOLOGISTS 1 NOV 2014, vol. 20, no. 11, 1 November 2014 (2014-11-01), pages 1187-1197, XP009185547, ISSN: 1934-2403
- None

## Description

### FIELD OF THE INVENTION

The present specification discloses methods for avoiding an increase in glucagon secretion associated with the administration of a sodium glucose co-transporter 2 (SGLT2) inhibitor via the co-administration of a dipeptidyl peptidase IV (DPP IV) inhibitor. The present specification also discloses methods for normalizing the glucagon secretion associated with the administration of a sodium glucose co-transporter 2 (SGLT2) inhibitor via the co-administration of a dipeptidyl peptidase IV (DPP IV) inhibitor. Furthermore, the present specification relates to methods for treating diabetes, both Type 1 and Type 2 diabetes, especially Type 2 diabetes, as well as hyperglycemia, hyperinsulinemia, obesity, hypertriglyceridemia, Syndrome X, diabetic complications, atherosclerosis and related diseases, comprising administering an SGLT2 inhibitor and a dipeptidyl peptidase IV (DPP IV) inhibitor.

### BACKGROUND OF THE INVENTION

SGLT2 is a member of a family of proteins that utilizes an electrochemical sodium gradient to transport glucose, against the sodium concentration gradient inside the cells. Different Na+/Glucose transporters are found in different tissues: SGLT1 is mainly found in intestinal mucosa in the small intestine and the S3 segment of the proximal tubule of the nephron in the kidney; and SGLT2 is mainly found in the S1 segment of the proximal tubule of the nephron in the kidney.

Diabetes Mellitus results from a mismatch between the demand and the supply of insulin resulting in increased glucose levels. Treatment of Diabetes Mellitus using SGLT2 inhibitors reduces plasma glucose by inhibiting the reabsorbtion of glucose in the kidneys, hence leading to an increase glucose excretion in the urine. In papers from DeFronzo and Ferranini it is shown that treatment with SGLT2 inhibitors lead to increase in glucagon secretion. The mechanism for the SGLT2 inhibitor stimulation of glucagon secretion is not known. Glucagon stimulates hepatic glucose production which counteracts the glucose lowering effect of SGLT2 inhibitors.

The current disclosure demonstrates that the increase in glucagon secretion secondary to SGLT2 inhibitor administration, surprisingly, can be avoided, normalized, and even counteracted (without effecting the insulin secretion from beta cells) using a dipeptidyl peptidase IV (DPP IV) inhibitor, for example saxagliptin. Additionally, said avoidance, normalization and counteraction surprisingly occur without any increased risk of hypoglycemia, impairment to respond to hypoglycemia and with body weight reduction. It is therefore believed that administering an SGLT2 inhibitor with a dipeptidyl peptidase IV (DPP IV) inhibitor, for example saxagliptin, will provide enhanced glycemic control.

### SUMMARY OF THE INVENTION

In one aspect, the present invention provides dapagliflozin for co-administration with saxagliptin for use in the treatment of diabetes, hyperglycemia, hyperinsulinemia, obesity, hypertriglyceridemia, Syndrome X, diabetic complications or atherosclerosis, wherein the combination normalizes glucagon secretion in a mammal associated with the administration of dapagliflozin.

In another aspect, the present invention provides dapagliflozin for co-administration with saxagliptin for use in the treatment of diabetes, hyperglycemia, hyperinsulinemia, obesity, hypertriglyceridemia, Syndrome X, diabetic complications or atherosclerosis, wherein the combination reduces the hepatic glucagose production in a mammal associated with the administration of dapagliflozin.

In yet another aspect, the present invention provides dapagliflozin for co-adminstration with saxagliptin for use in the treatment of diabetes, hyperglycemia, hyperinsulinemia, obesity, hypertriglyceridemia, Syndrome X, diabetic complications or atherosclerosis, wherein the combination improves glycemic control in a mammal via an avoidance in an increase in glucagon secretion associated with the administration of dapagliflozin.

In a further aspect, the present specification discloses methods for treating diabetes, especially Type 2 diabetes, in a mammal comprising co-administering to the mammal in need of such treatment a therapeutically effective amount of an SGLT2 inhibitor and a dipeptidyl peptidase IV (DPP IV) inhibitor.

Specific preferred embodiments of the present invention will become evident from the following more detailed description of certain preferred embodiments and the claims.

### DETAILED DESCRIPTION OF THE INVENTION

Described herein are methods for avoiding an increase in glucagon secretion associated with the administration of a sodium glucose co-transporter 2 (SGLT2) inhibitor or a pharmaceutically acceptable salt thereof, a stereoisomer thereof, or a prodrug ester thereof, comprising administering the SGLT2 inhibitor and a dipeptidyl peptidase IV (DPP IV) inhibitor.

Also described herein are methods for normalizing glucagon secretion associated with the administration of a sodium glucose co-transporter 2 (SGLT2) inhibitor or a pharmaceutically acceptable salt thereof, a stereoisomer thereof, or a prodrug ester thereof, comprising administering the SGLT2 inhibitor and a dipeptidyl peptidase IV (DPP IV) inhibitor.

Also described herein are methods of reducing hepatic glucose production comprising administering a sodium glucose co-transporter 2 (SGLT2) inhibitor or a pharmaceutically acceptable salt thereof, a stereoisomer thereof, or a prodrug ester thereof, and a dipeptidyl peptidase IV (DPP IV) inhibitor.

Also described herein are methods of improving glycemic control in a mammal via an avoidance in an increase in glucagon secretion comprising co-administering to the mammal in need of such control a therapeutically effective amount of a sodium glucose co-transporter 2 (SGLT2) inhibitor or a pharmaceutically acceptable salt thereof, a stereoisomer thereof, or a prodrug ester thereof, and a dipeptidyl peptidase IV (DPP IV) inhibitor.

Also described herein are methods for treating diabetes, preferably Type 1 and Type 2 diabetes, more preferably Type 2 diabetes, in a mammal via an avoidance in an increase in glucagon secretion comprising co-administering to the mammal in need of such treatment a therapeutically effective amount of a sodium glucose co-transporter 2 (SGLT2) inhibitor or a pharmaceutically acceptable salt thereof, a stereoisomer thereof, or a prodrug ester thereof, and a dipeptidyl peptidase IV (DPP IV) inhibitor.

Also described herein are methods wherein the mammal is a human.

The SGLT2 inhibitors described are most preferably selective for SGLT2 relative to SGLT1. High selectivity for SGLT2, as is the case with dapagliflozin, is advantageous because it avoids the unpredictable effects of intestinal SGLT1 inhibition.

Selectivity for SGLT2 of a given inhibitor can be determined by comparing the EC₅₀ values measured in an SGLT1 and SGLT2 assay. Briefly, Human SGLT1 (hSGLT1) and human SGLT2 (hSGLT2) full-length cDNA sequences are cloned by PCR using MARATHON READY™ human kidney cDNA (Clontech, Mountain View, CA), with primers designed from published sequences (Genbank accession numbers NM_003041 and NM_000343). The hSGLT1 and hSGLT2 sequences are cloned into pIRESneo vector (Clontech, Mountain View, CA) for mammalian expression and are stably transfected into Chinese hamster ovary (CHO) cells. SGLT-expressing clones are selected based on resistance to G418 antibiotic (GENETICIN®, Invitrogen, Carlsbad, CA) and activity in the ¹⁴C-α-methyl-D-glucopyranoside (¹⁴C-AMG) uptake assay.

Cells expressing hSGLT1 or hSGLT2 are maintained using standard cell culture techniques. Assays for sodium-dependent glucose transport in 96-well plates are initiated by adding 100 µl/well of protein-free assay buffer containing sodium (Hepes/Tris pH 7.4, 137 mM NaCl, 5.4 mM KCl, 2.8 mM CaCl₂, 1.2 mM MgSO₄), 10 µM ¹⁴C-AMG and inhibitor or dimethyl sulfoxide (DMSO) vehicle, and plates are incubated for 2 h at 37°C. Sodium-dependent ¹⁴C-AMG uptake is calculated by subtracting the counts per minute (CPM) observed under sodium-free uptake conditions from the counts observed under sodium-containing conditions. Inhibitors are assayed at various concentrations in triplicate in the presence of sodium, and the percent inhibition is calculated by comparing CPM in inhibitor-containing wells with CPM in wells containing only DMSO vehicle. Phlorizin, a known SGLT inhibitor, is evaluated in parallel in every assay. A dose-response curve is fitted to an empirical four-parameter model using XL Fit (IDBS, Guilford, UK) to determine the inhibitor concentration at half-maximal response (EC₅₀). SGLT2 selectivity is represented as a ratio of EC₅₀ in favor of SGLT2. An SGLT2 inhibitor with an EC₅₀ selective ratio of at least 10, and more preferably at least 100, in favor of SGLT2 is suitable for use in the instant invention.

SGLT2 inhibitors suitable for use in accordance with the description comprise C-arylglucosides or O-arylglucosides. C-arylglucosides and O-arylglucosides are effective in treating diabetes. See U.S. Patent No. 6,774,112, which is incorporated herein by reference in its entirety.

Examples of C-arylglucoside (also referred to as C-glucosides) SGLT2 inhibitors, include, but are not limited to, the following:
1) C-aryl glucosides as disclosed in U.S. Patent Nos. 6,515,117 and 6,414,126;
2) C-aryl glucosides as described in U.S. Patent Application No. 11/233617 (U.S. Patent Application Publication No. 2006/0063722 A1);
3) C-aryl glucosides described in U.S. Patent No. 6,774,112;
4) Glucopyranosyl-substituted benzene derivatives as disclosed in U.S. Patent Application Publication No. 2005/0209166; and
5) D-pyranosyl-substituted phenyl compounds as disclosed in U.S. Patent Application Publication No. S 2006/0074031.

Examples of O-glucoside SGLT2 inhibitors include, but are not limited to, those described in the following:
1) 5-Thio-β-D-glucopyranoside as disclosed in U.S. Patent Application Publication No. 2006/0194809;
2) Glucopyranyloxybenzene derivatives as disclosed in WO 03/01180;
3) Pyrazole derivatives as disclosed in U.S. Patent No. 6,908,905; and
4) Pyrazole compounds as disclosed in U.S. Patent No. 6,815,428.

Other disclosures and publications disclosing SGLT2 inhibitors are as follows: K. Tsujihara et al., Chem. Pharm. Bull., 44:1174-1180 (1996); M. Hongu et al., Chem. Pharm. Bull., 46:22-33 (1998); M. Hongu et al., Chem. Pharm. Bull., 46:1545-1555 (1998); A. Oku et al., Diabetes, 48:1794-1800 (1999); and JP 10245391 (Dainippon).

In a preferred aspect the SGLT2 inhibitor is compound I or dapagliflozin, as disclosed in U.S. Patent No. 6,515,117 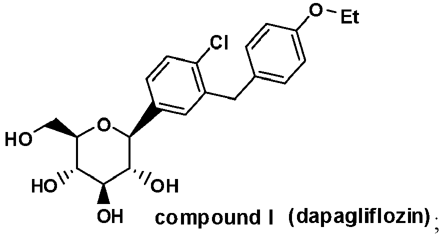 or a pharmaceutically acceptable salt thereof, all stereoisomers thereof, or a prodrug ester thereof.

In another preferred aspect, the invention provides crystalline forms of compound I including the crystalline forms disclosed in U.S. Patent Application Publication No. 2008/0004336. Most preferred crystalline forms for use in the methods of the present invention are dapagliflozin (S) propylene glycol hydrate and dapagliflozin (R) propylene glycol hydrate.

Additional SGLT2 inhibitors include canagliflozin (Johnson & Johnson/Mitsubishi Tanabe Pharma); remogliflozin etabonate (Glaxo Smithkline Pic, Kissei Pharmaceuticals Co.); ipragliflozin (Astellas/Kotobuki); empagliflozin (Boehringer Ingelheim); BI-44847 (Boehringer Ingelheim); TS-071 (Taisho Pharmaceutical); tofogliflozin (Roche/Chugai Pharmaceutical); LX-4211 (Lexicon Pharmaceuticals); DSP-3235 (GlaxoSmithKline/Dainippon Sumitomo); ISIS-SGLT2Rx (Isis Pharmaceuticals); and YM543 (Astellas Pharma Inc).

Examples of DPP-IV inhibitors include, but are not limited to, saxaglipitn, sitagliptin (Merck & Co., Inc., Ono Pharmaceuticals Co., Ltd); vildagliptin (Novartis Pharma AG); linagliptin (Boehringer Ingelheim and Eli Lilly)); alogliptin (Takeda Pharmaceutical Company Ltd.), other known DPP-IV inhibitors or other antihyperglycemic agents which act on the ATP-dependent channel of the β-cells, with saxagliptin being preferred.

Methods wherein the DPP-IV inhibitor is linagliptin and SGLT2 inhibitor is empagliflozin are disclosed.

The SGLT2 inhibitors and DPP IV inhibitors employed in accordance with the description can be administered to various mammalian species, such as dogs, cats, cattle, humans, etc., in need of treatment. These agents can be administered systemically, such as orally or parenterally.

The present description provides for the co-administration of one or more additional anti-diabetic agents.

Examples of suitable additional anti-diabetic agents for use in co-administration of the present description include, but are not limited to, biguanides (e.g., metformin or phenformin), glucosidase inhibitors (e.g., acarbose or miglitol), insulins (including insulin secretagogues or insulin sensitizers), meglitinides (e.g., repaglinide), sulfonylureas (e.g., glimepiride, glyburide, gliclazide, chlorpropamide and glipizide), biguanide/glyburide combinations (e.g., Glucovance®), thiazolidinediones (e.g., troglitazone, rosiglitazone and pioglitazone), PPAR-alpha agonists, PPAR-gamma agonists, PPAR alpha/gamma dual agonists, glycogen phosphorylase inhibitors, inhibitors of fatty acid binding protein (aP2), glucagon-like peptide-1 (GLP-1) and other agonists of the GLP-1 receptor.

Other suitable thiazolidinediones include, but are not limited to, MCC-555 (disclosed in U.S. Patent No. 5,594,016, Mitsubishi), faraglitazar (GI-262570, Glaxo-Wellcome), englitazone (CP-68722, Pfizer), darglitazone (CP-86325, Pfizer); isaglitazone (MIT/Johnson& Johnson), reglitazar (JTT-501, (JPNT/Pharmacia & Upjohn), rivoglitazone (R-119702, Sankyo/WL), liraglutide (NN-2344, Dr. Reddy/NN), and (Z)-1,4-bis-4-[(3,5-dioxo-1,2,4-oxadiazolidin-2-yl-methyl)]phenoxybut-2-ene (YM-440, Yamanouchi).

Examples of PPAR-alpha agonists, PPAR-gamma agonists and PPAR alpha/gamma dual agonists include, but are not limited to, muraglitazar, peliglitazar, tesaglitazar AR-HO39242 (AstraZeneca), GW-501516 (Glaxo-Wellcome), KRP297 (Kyorin Merck), as well as those disclosed by Murakami et al, "A Novel Insulin Sensitizer Acts As a Coligand for Peroxisome Proliferation - Activated Receptor Alpha (PPAR alpha) and PPAR gamma. Effect on PPAR alpha Activation on Abnormal Lipid Metabolism in Liver of Zucker Fatty Rats", Diabetes 47, 1841-1847 (1998); WO 01/21602 and in U.S. Patent No. 6,414,002 and U.S Patent No. 6,653,314

Suitable aP2 inhibitors include, but are not limited to, those disclosed in U.S. application Serial No. 09/391,053, filed September 7, 1999, and in U.S. Patent No. 6,548,529.

Other suitable meglitinides include nateglinide (Novartis) or KAD1229 (PF/Kissei).

Also described herein is the co-administration of one or more additional anti-diabetic agents, wherein the additional anti-diabetic agent is metformin.

The SGLT2 inhibitors, DPP IV inhibitors and any additional anti-diabetic agents utilized in the present description are administered as pharmaceutical compositions that comprise the compounds formulated together with one or more pharmaceutically acceptable carriers. The pharmaceutical compositions can be specially formulated for oral administration in solid or liquid form, for parenteral injection or for rectal administration.

Pharmaceutical compositions can be administered to humans and other mammals orally, rectally, parenterally, intracisternally, intravaginally, intraperitoneally, bucally or as an oral or nasal spray. The term "parenterally," as used herein, refers to modes of administration which include intravenous, intramuscular, intraperitoneal, intrasternal, subcutaneous and intraarticular injection and infusion.

The SGLT2 inhibitors, DPP IV inhibitors and any additional anti-diabetic agents are incorporated in a conventional systemic dosage form, such as a tablet, capsule, elixir or injectable formulation. The above dosage forms will also include the necessary physiologically acceptable carrier material, excipient, lubricant, buffer, antibacterial, bulking agent (such as mannitol), antioxidants (ascorbic acid or sodium bisulfite) or the like. Oral dosage forms are preferred, although parenteral forms are quite satisfactory as well.

Pharmaceutical compositions for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), vegetable oils (such as olive oil), injectable organic esters (such as ethyl oleate) and suitable mixtures thereof. Proper fluidity can be maintained, for example, by the use of coating materials such as lecithin, by the maintenance of the required particle size in the case of dispersions and by the use of surfactants.

These compositions may also contain adjuvants such as preservatives, wetting agents, emulsifying agents and dispersing agents. Prevention of the action of microorganisms can be ensured by the inclusion of various antibacterial and antifungal agents, for example, paraben, chlorobutanol, phenol sorbic acid and the like. It may also be desirable to include isotonic agents such as sugars, sodium chloride and the like. Prolonged absorption of the injectable pharmaceutical form can be brought about by the inclusion of agents which delay absorption such as aluminum monostearate and gelatin.

In some cases, in order to prolong the effect of the drug, it is desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

Injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Solid dosage forms for oral administration include capsules, tablets, pills, powders and granules. In such solid dosage forms, the active compound is mixed with at least one inert, pharmaceutically acceptable excipient or carrier, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

Solid dosage forms of tablets, dragees, capsules, pills and granules can be prepared with coatings and shells such as enteric coatings and other coatings well-known in the pharmaceutical formulating art. They may optionally contain opacifying agents and may also be of a composition such that they release the active ingredient(s) only, or preferentially, in a certain part of the intestinal tract, optionally, in a delayed manner. Examples of embedding compositions which can be used include polymeric substances and waxes.

The SGLT2 inhibitors, DPP IV inhibitors and any additional anti-diabetic agents can also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, tragacanth and mixtures thereof.

The dose administered is adjusted according to the age, weight, and condition of the patient, as well as the route of administration, dosage form and regimen, and the desired result. In general, the dosage forms described above can be administered containing amounts of SGLT2 inhibitor of from about 1 to about 1000 mg per day, preferably from about 2 to about 400 mg per day, more preferably 2.5 to about 75 mg/day, and even more preferably from 2.5 to about 50 mg/day.

The dosage forms can be administered in single or divided doses of one to four times daily.

Unless otherwise indicated, dosages and formulations for SGLT2 inhibitors, DPP IV inhibitors and any additional anti-diabetic agents used in the methods set forth herein are disclosed in the various patents and applications discussed throughout the application, which are incorporated herein in their entireties.

It will be recognized by one of skill in the art that the amount of drug required for therapeutic effect on administration will, of course, vary with the agent chosen, the nature and severity of the condition and the mammal undergoing treatment, and is ultimately at the discretion of the physician. Furthermore, the optimal quantity and spacing of individual dosages of a drug will be determined by the nature and extent of the therapeutic effects desired, the form, route and site of administration, the particular patient being treated and that such optima can be determined by conventional techniques. It will also be appreciated that the optimal course of treatment, for example, the number of doses given, can be ascertained by those skilled in the art using conventional course of treatment determination tests.

SGLT2 inhibitor activity of the compounds useful in the present description can be determined by use of an assay system as set out below.

### ASSAY FOR SGLT2 ACTIVITY

The mRNA sequence for human SGLT2 is cloned by reverse-transcription and amplification from human kidney mRNA, using standard molecular biology techniques. The cDNA sequence is stably transfected into CHO cells, and clones are assayed for SGLT2 activity essentially as described in Ryan et al., 1994, "HK-2: an immortalized proximal tubule epithelial cell line from normal adult human kidney," Kidney International 45:48-57. Evaluation of inhibition of SGLT2 activity in a clonally selected cell line is performed essentially as described in Ryan *et al.,* with the following modifications. Cells are grown in 96-well plates for 2-4 days to 75,000 or 30,000 cells per well in F-12 nutrient mixture (Ham's F-12; GIBCO, Long Island, NY), 10% fetal bovine serum, 300 µg/ml Geneticin and penicillin-streptomycin. At confluence, cells are washed twice with 10 mM Hepes/Tris, pH 7.4, 137 mM N-methyl-D-glucamine, 5.4 mM KCl, 2.8 mM CaCl₂, 1.2 mM MgSO₄. Cells then are incubated with 10 µM [¹⁴C]AMG, and 10 µM inhibitor (final DMSO =0.5%) in 10 mM Hepes/Tris, pH 7.4, 137 mM NaCl, 5.4 mM KCl, 2.8 mM CaCl₂, 1.2 mM MgSO₄ at 37°C for 1.5 hr. Uptake assays are quenched with ice cold IX PBS containing 0.5 mM phlorizin, and cells are then lysed with 0.1% NaOH. After addition of MicroScint scintillation fluid, the cells are allowed to shake for 1 hour, and then [¹⁴C]AMG is quantified on a TopCount scintillation counter. Controls are performed with and without NaCl. For determination of EC₅₀ values, 10 inhibitor concentrations are used over 2 log intervals in the appropriate response range, and triplicate plates are averaged across plates. Ryan *et al., Id.*

### EXAMPLE

The following is illustrative of the present invention.

Adult patients with type 2 diabetes mellitus (T2DM) and glycated hemoglobin (HbA1c) levels greater than or equal to 8% and less than or equal to 12% at screening who were on stable metformin therapy for at least 8 weeks were randomized 1:1:1 to receive either a saxagliptin (5 mg/day) and dapagliflozin (10 mg/day) plus metformin XR (1500-2000 mg/day) dosage, a saxagliptin (5 mg/day) and placebo plus metformin XR (1500-2000 mg/day) dosage, or a dapagliflozin (10 mg/day) and placebo plus metformin XR (1500-2000 mg/day) dosage for 24 weeks.

At the beginning of a 4-week lead-in period, all patients were switched to the nearest metformin XR dose (1,500-2,000 mg/day) for the lead-in period and for the duration of the 24-week treatment period. Patients were then randomized to receive saxagliptin 5 mg/day and dapagliflozin 10 mg/day plus metformin (SAXA + DAPA + MET), saxagliptin 5 mg/day and placebo plus metformin (SAXA + MET), or dapagliflozin 10 mg/day and placebo plus metformin (DAPA + MET) for 24 weeks. Patients were prohibited from receiving other antidiabetic medications (except for open-label rescue medications) during the screening and treatment periods.

Postprandial glucose (PPG) was assessed at baseline and week 24 following the administration of a liquid meal tolerance test (MTT, 360-375 kcal; protein, 14-28.2 g; fat, 10.5-14 g; carbohydrates 42-45 g; sugars as a component of carbohydrates, 16.8-22 g, investigation-site dependent). The MTT was conducted before drug administration at baseline and 1 hour after drug administration at 24 weeks. Blood samples were drawn for the determination of glucagon at time 0, 30, 60, 120, and 180 minutes. The mean change from baseline in the area under the curve from 0 to 180 minutes (AUC_{0-180 min}) was calculated for glucagon obtained during the MTT. Plasma glucagon was measured by radioimmunoassay (LINCO Research, St. Charles, MO).

AUC_{0-180 min} was calculated based on the trapezoidal rule from model-based estimates of the mean at each time point, including observations prior to rescue. Analyses of the mean changes from baseline at 24 weeks in AUC_{0-180 min} for glucagon obtained during the MTT were performed using a longitudinal repeated-measures analysis with terms for baseline value, treatment group, time, interaction of treatment group and time, and interaction of baseline value and time. Point estimates and 95% confidence intervals (CIs) were calculated for the adjusted mean changes within each treatment group, as well as for the differences in adjusted mean changes between treatment groups. To assess the correlation of changes in HbAlc versus changes in AUC_{0-180 min} for glucagon at 24 weeks, changes in HbAlc versus changes in fasting glucagon concentrations, and baseline fasting glucagon concentrations or glucagon AUC_{0-180 min} with baseline HbAlC posthoc regression analyses were performed. The regression model included fixed effects of treatment and changes in AUC_{0-180 min} for glucagon at 24 weeks, fasting glucagon concentrations, baseline fasting glucagon concentrations, or glucagon AUC_{0-180 min}. No multiplicity control was applied. The data is set forth in Table 1 below.

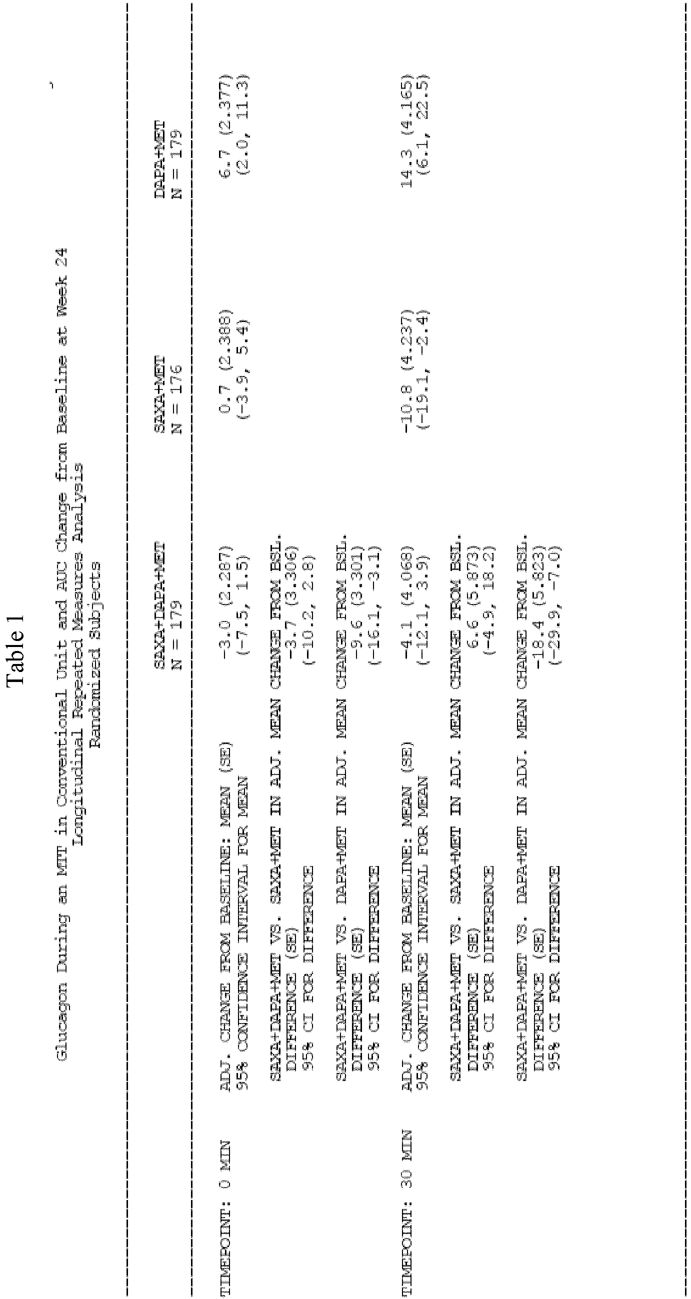

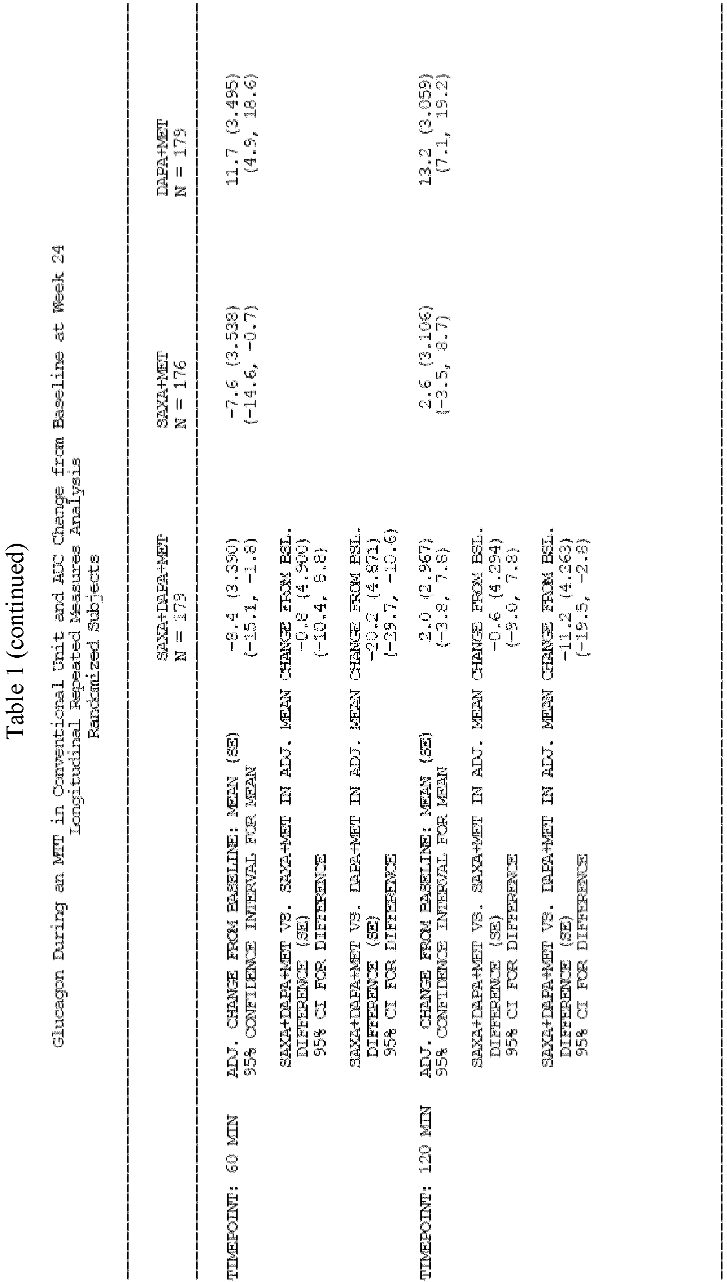

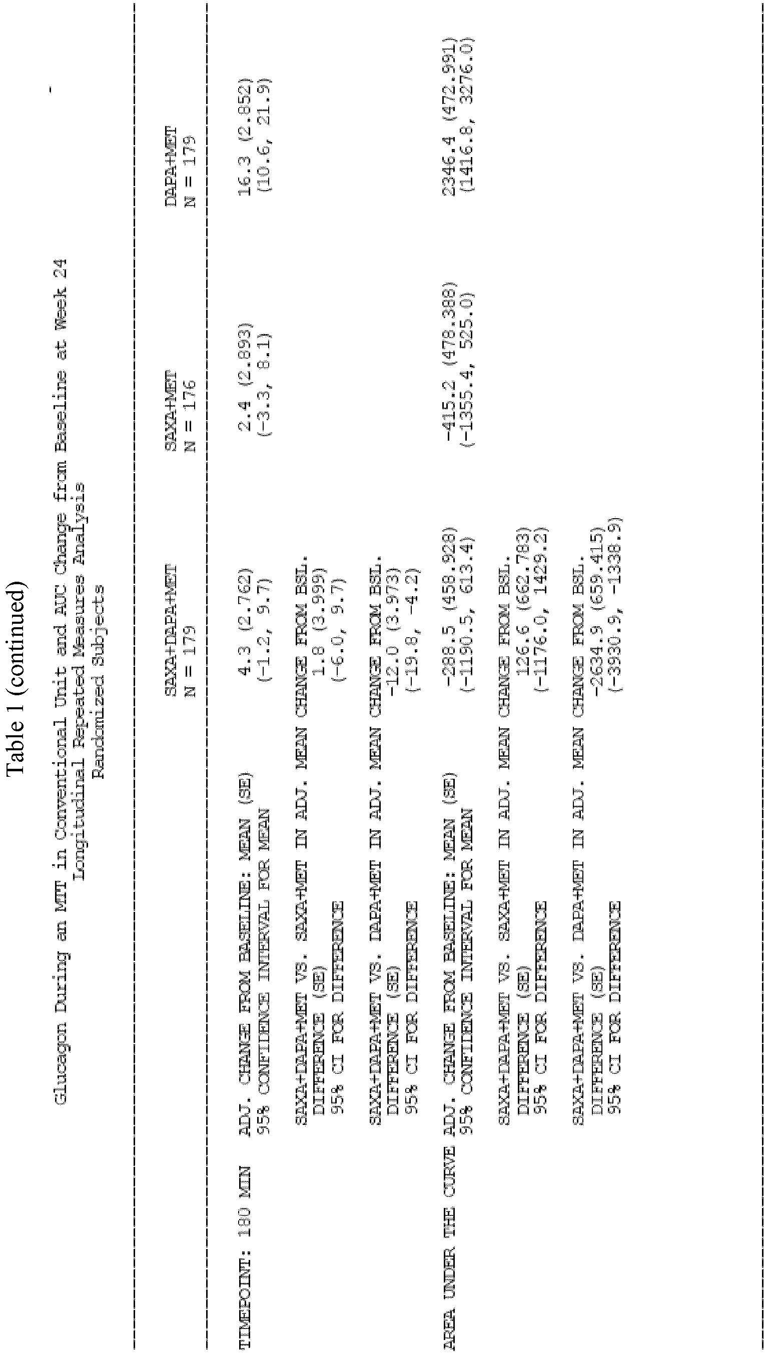

The data set forth in Table 1 demonstrates that the glucagon levels are increased compared to control when dapagliflozin was added and that this effect was counteracted, and unexpectedly completely eliminated, by the addition of saxagliptin.

In view of these findings, it is believed that the co-administration of an SGLT2 inhibitor and a DPP IV inhibitor, and optionally an additional anti-diabetic agent, like metformin, will be effective in controlling glycemic levels and treating diabetes, especially Type 2 diabetes, as well as hyperglycemia, hyperinsulinemia, obesity, hypertriglyceridemia, Syndrome X, diabetic complications, atherosclerosis and related diseases, in mammals.

## Claims

1. Dapagliflozin for co-administration with saxagliptin for use in the treatment of diabetes, hyperglycemia, hyperinsulinemia, obesity, hypertriglyceridemia, Syndrome X, diabetic complications or atherosclerosis, wherein the combination normalizes glucagon secretion in a mammal associated with the administration of dapagliflozin.

2. Dapagliflozin for co-administration with saxagliptin for use in the treatment of diabetes, hyperglycemia, hyperinsulinemia, obesity, hypertriglyceridemia, Syndrome X, diabetic complications or atherosclerosis, wherein the combination reduces the hepatic glucagose production in a mammal associated with the administration of dapagliflozin.

3. Dapagliflozin for co-administration with saxagliptin for use in the treatment of diabetes, hyperglycemia, hyperinsulinemia, obesity, hypertriglyceridemia, Syndrome X, diabetic complications or atherosclerosis, wherein the combination improves glycemic control in a mammal via an avoidance in an increase in glucagon secretion associated with the administration of dapagliflozin.

4. Dapagliflozin for use according to any preceding claim, wherein the diabetes is Type 2 diabetes.

5. Dapagliflozin for use according to any of claims 1 to 4, wherein the mammal is a human.

6. Dapagliflozin for use according to any of claims 1 to 5, further comprising the co-administration of one or more additional anti-diabetic agents.

7. Dapagliflozin for use according to claim 6, wherein the one or more additional anti-diabetic agents are selected from the group consisting of a biguanide, a glucosidase inhibitor, insulin, a meglitinide, a sulfonylurea, a biguanide/glyburide combination, a thiazolidinedione, a PPAR-alpha agonist, a PPAR-gamma agonist, a PPAR alpha/gamma dual agonist, a glycogen phosphorylase inhibitor, an inhibitor of fatty acid binding protein (aP2), a glucagon-like peptide-1 (GLP-1) and another agonist of the GLP-1 receptor.

8. Dapagliflozin for use according to claim 6, wherein the one or more additional anti-diabetic agent is metformin.

## Patentansprüche

1. Dapagliflozin zur gemeinsamen Verabreichung mit Saxagliptin zur Verwendung bei der Behandlung von Diabetes, Hyperglykämie, Hyperinsulinämie, Fettleibigkeit, Hypertriglyceridämie, Syndrom X, Diabeteskomplikationen oder Atherosklerose, wobei durch die Kombination die Glucagonsekretion bei einem Säuger in Zusammenhang mit der Verabreichung von Dapagliflozin normalisiert wird.

2. Dapagliflozin zur gemeinsamen Verabreichung mit Saxagliptin zur Verwendung bei der Behandlung von Diabetes, Hyperglykämie, Hyperinsulinämie, Fettleibigkeit, Hypertriglyceridämie, Syndrom X, Diabeteskomplikationen oder Atherosklerose, wobei durch die Kombination die Glucagoseproduktion in der Leber bei einem Säuger in Zusammenhang mit der Verabreichung von Dapagliflozin reduziert wird.

3. Dapagliflozin zur gemeinsamen Verabreichung mit Saxagliptin zur Verwendung bei der Behandlung von Diabetes, Hyperglykämie, Hyperinsulinämie, Fettleibigkeit, Hypertriglyceridämie, Syndrom X, Diabeteskomplikationen oder Atherosklerose, wobei durch die Kombination die glykämische Kontrolle bei einem Säuger über eine Vermeidung eines Anstiegs der Glucagonsekretion in Zusammenhang mit der Verabreichung von Dapagliflozin verbessert wird.

4. Dapagliflozin zur Verwendung nach einem vorhergehenden Anspruch, wobei es sich bei dem Diabetes um Typ-2-Diabetes handelt.

5. Dapagliflozin zur Verwendung nach einem der Ansprüche 1 bis 4, wobei es sich bei dem Säuger um einen Menschen handelt.

6. Dapagliflozin zur Verwendung nach einem der Ansprüche 1 bis 5, ferner umfassend die gemeinsame Verabreichung eines oder mehrerer zusätzlicher Antidiabetika.

7. Dapagliflozin zur Verwendung nach Anspruch 6, wobei die ein oder mehreren zusätzlichen Antidiabetika ausgewählt sind aus der Gruppe bestehend aus einem Biguanid, einem Glukosidasehemmer, Insulin, einem Meglitinid, einem Sulfonylharnstoff, einer Biguanid/Glyburid-Kombination, einem Thiazolidindion, einem PPAR-alpha-Agonisten, einem PPAR-gamma-Agonisten, einem PPAR-alpha/gamma-Doppelagonisten, einem Glycogenphosphorylase-Inhibitor, einem Inhibitor von Fettsäure-Bindungsprotein (aP2), einem GLP-1 (Glucagon-like Peptide-1) und einem weiteren Agonisten des GLP-1-Rezeptors.

8. Dapagliflozin zur Verwendung nach Anspruch 6, wobei es sich bei dem mindestens einen zusätzlichen Antidiabetikum um Metformin handelt.

## Revendications

1. Dapagliflozine pour une co-administration avec de la saxagliptine pour une utilisation dans le traitement du diabète, de l'hyperglycémie, de l'hyperinsulinémie, de l'obésité, de l'hypertriglycéridémie, du Syndrome X, de complications diabétiques ou de l'athérosclérose, la combinaison normalisant la sécrétion de glucagon chez un mammifère associé à l'administration de dapagliflozine.

2. Dapagliflozine pour une co-administration avec de la saxagliptine pour une utilisation dans le traitement du diabète, de l'hyperglycémie, de l'hyperinsulinémie, de l'obésité, de l'hypertriglycéridémie, du Syndrome X, de complications diabétiques ou de l'athérosclérose, la combinaison réduisant la production de glucagose hépatique chez un mammifère associé à l'administration de dapagliflozine.

3. Dapagliflozine pour une co-administration avec de la saxagliptine pour une utilisation dans le traitement du diabète, de l'hyperglycémie, de l'hyperinsulinémie, de l'obésité, de l'hypertriglycéridémie, du Syndrome X, de complications diabétiques ou de l'athérosclérose, la combinaison améliorant le contrôle glycémique chez un mammifère via un évitement d'une augmentation de la sécrétion de glucagon associé à l'administration de dapagliflozine.

4. Dapagliflozine pour une utilisation selon une quelconque revendication précédente, le diabète étant un diabète de Type 2.

5. Dapagliflozine pour une utilisation selon l'une quelconque des revendications 1 à 4, le mammifère étant un humain.

6. Dapagliflozine pour une utilisation selon l'une quelconque des revendications 1 à 5, comprenant en outre la co-administration d'un ou plusieurs agents antidiabétiques supplémentaires.

7. Dapagliflozine pour une utilisation selon la revendication 6, l'agent ou les agents antidiabétiques supplémentaires étant choisis dans le groupe constitué par un biguanide, un inhibiteur de glucosidase, l'insuline, un méglitinide, une sulfonylurée, une combinaison biguanide/glyburide, une thiazolidinedione, un agoniste de PPAR-alpha, un agoniste de PPAR-gamma, un agoniste dual PPAR alpha/gamma, un inhibiteur de glycogène phosphorylase, un inhibiteur de protéine de liaison d'acide gras (aP2), un glucagon-like peptide-1 (GLP-1) et un autre agoniste du récepteur de GLP-1.

8. Dapagliflozine pour une utilisation selon la revendication 6, l'agent ou les agents antidiabétiques supplémentaires étant la metformine.
